(19) Europäisches Patentamt
European Patent Office
Office européen des brevets

(11) Publication number: 0 442 576 A1

# (12) EUROPEAN PATENT APPLICATION

(21) Application number: 91200287.0

(22) Date of filing: 11.02.91

(51) Int. Cl.5: C07F 7/10, C07D 205/085

(30) Priority: 13.02.90 EP 90200341

(43) Date of publication of application:
21.08.91 Bulletin 91/34

(84) Designated Contracting States:
AT BE CH DE DK ES FR GB GR IT LI LU NL SE

(71) Applicant: RIJKSUNIVERSITEIT UTRECHT
Padualaan 8
NL-3584 CH Utrecht(NL)

(72) Inventor: van Koten, Gerard
Paltzerweg 180
NL-3734 DC Den Dolder(NL)
Inventor: van der Steen, Frederik Hendrik
Huetlaan 11
NL-3553 GN Utrecht(NL)
Inventor: Jastrzebski, Johann Tonns Barthold Heinrich
Stratusplein 11
NL-3731 XA De Bilt(NL)
Inventor: Kleijn, Henk
Trekvalk 10
NL-3435 ZP Nieuwegein(NL)

(74) Representative: Matulewicz, Emil Rudolf Antonius, Dr. et al
Gist-Brocades NV Patents and Trademarks Department Wateringseweg 1 P.O. Box 1
NL-2600 MA Delft(NL)

## (54) Synthesis of cis-beta-lactams with the aid of an alkali metal compound.

(57) A synthesis of <u>cis</u>-$\beta$-lactams in a nearly quantitative yield by a condensation reaction of an alkali metal enolate, a substituted glycine ester and an alkali metal base, with an activated imine, is provided.

EP 0 442 576 A1

## SYNTHESIS OF CIS-$\beta$-LACTAMS WITH THE AID OF AN ALKALI METAL COMPOUND

The present invention relates to the synthesis of cis-$\beta$-lactam compounds and to new intermediates therefor.

As a result of the constant need for new and more efficacious antibiotics, partly due to the appearance of strains of bacteria that are genetically resistant to many commonly used antibiotics, considerable chemical research effort has been and will be expended on the development of both new and known antibiotics. The azetidinones or $\beta$-lactams have received the most attention in recent years since they are generally well tolerated by human beings.

In EP-A-281177 a synthetic route is described to synthesize 1,3,4-trisubstituted trans-2-azetidinones in which the atom numbering conventionally is as follows:

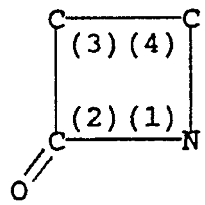

A process starting from a metal enolate (II'), an alkali metal base (P-base) and a metal compound (VIII'), and a one-pot process starting from an $R_2'''R_3'''$N-glycine ester (V'), both involving reaction with an imine (IV'), are described in EP-A-281177 (see the following scheme I):

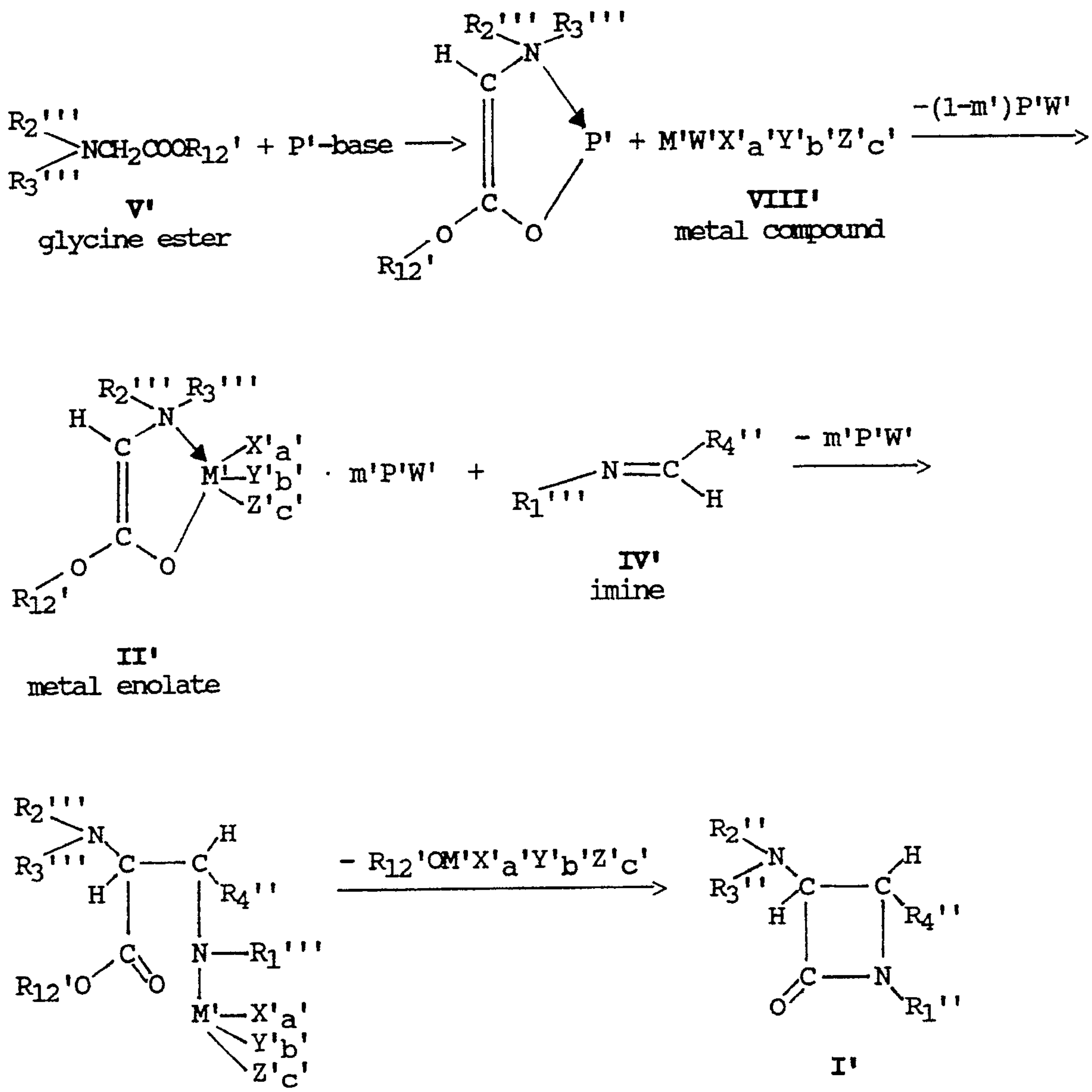


V' glycine ester

VIII' metal compound

II' metal enolate

IV' imine

I'

**Scheme I**

wherein

$R_2$" and $R_3$" are each, independently, hydrogen, alkyl, aryl or aralkyl, each optionally substituted by an alkyl, aryl or aralkyl group, with the proviso that they are not both methyl or both benzyl; or are

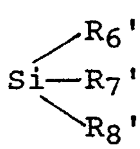

where $R_6$', $R_7$' and $R_8$' are each alkyl, aryl or aralkyl, each optionally substituted and $R_6$', $R_7$' and $R_8$' are the same or different;

or $R_2$" and $R_3$", together with the nitrogen atom to which they are attached, form a ring with up to 8 ring atoms, each ring atom optionally substituted by an alkyl, aryl or aralkyl group;

$R_1$" is hydrogen, halogen, or an alkyl, alkenyl, alkoxy, aryl or aralkyl group, or is

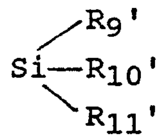

where $R_9'$, $R_{10}'$ and $R_{11}'$ are each, independently, an alkyl, aryl or aralkyl group, each optionally substituted;

$R_4''$ is an alkyl, hydroxy, halogen, sulphonyl, alkoxy, alkenyl, alkynyl or aryl group, each optionally substituted, or is

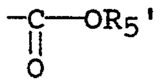

where $R_5'$ is an alkyl group; $R_{12}'$ is an alkyl, aryl or aralkyl group, each optionally substituted by an alkyl, aryl or aralkyl group;

M' is zinc, aluminum, zirconium, boron, tin or titanium;

P' is an alkali metal;

W', X', Y' and Z' are each, independently, an alkyl, aryl, halide, alkoxide, thiolate, triflate or other substituted sulphonate or other anionic group;

and a', b', c' and m' are each from 0 to 1 with a', b', c' being integers;

$R_2'''$ and $R_3'''$ are as defined above for $R_2''$ and $R_3''$, respectively, with the proviso that when $R_2''$ and $R_3''$ are hydrogen, $R_2'''$ and $R_3'''$ form, together with the nitrogen atom to which they are attached, a ring of formula III'

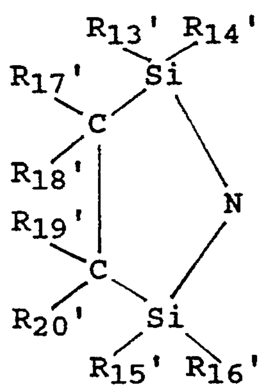

III'

wherein

$R_{13}'$, $R_{14}'$, $R_{15}'$, $R_{16}'$, $R_{17}'$, $R_{18}'$, $R_{19}'$ and $R_{20}'$ are each, independently, an alkyl, aryl or aralkyl group, which group is subjected to acid or base hydrolysis after the reaction;

$R_1'''$ is as defined above for $R_1''$, with the proviso that when $R_1''$ is hydrogen, $R_1'''$ is

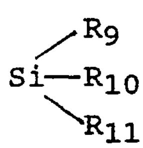

where $R_9$, $R_{10}$ and $R_{11}$ are as defined above,

which group is subjected to acid or base hydrolysis after the reaction.

In the non-prepublished EP-A-404267 similar reactions involving reactions of metal enolates with an imine with a heterocyclic group or with a diimine yielding enantioselective trans-β-lactams are disclosed. The non-prepublished EP-A-357118 disclosed that also trans-β-lactams are formed if the reactions are carried out in the presence of only catalytic amounts of the suitable metal compound MW(X)a(Y)b(Z)c. However, without such a metal compound, no β-lactam compound was formed at all.

The use of lithium enolates (formed in the reaction mixture without said metal compound) for the preparation of azetidinones has been described earlier, viz.:

- P. Andreoli c.s. (J. Chem. Soc. Perkin Trans. I, 945 (1988) and Tetrahedron Letters 27 (15), 1695 (1986)) and G. Cainelli c.s. (Tetrahedron Letters, 28 (44), 5369 (1987)) described the reaction of a lithium ester enolate with an adduct, obtained from the reduction of a nitrile with an aluminum hydride and chlorotrimethylsilane. The cis-β-lactams were formed in a yield of 40-70% and a stereoselectivity varying from 0% (cis:trans = 50:50) and 90% (cis:trans = 95:5);
- L. Overman c.s. (European Patent Application No. 180398 and J. Amer. Chem. Soc., 107, 1698 (1985)) disclosed the reaction of a lithium ester enolate with N-(cyanomethyl)amine affording β-lactams in a yield of < 81% (no stereoselectivity mentioned).

It has now surprisingly been found that carrying out the reactions as indicated in Scheme I without the metal compound $MW(X)_a(Y)_b(Z)_c$, but with the use of a suitable, activated imine, cis-β-lactams are formed in quantitative yields (> 90%) with a high stereoselectivity (the enantiomeric excess varying from about 60% to 99%). Particularly the yield of the cis-β-lactams formed has been significantly increased by applying the process according to the present invention. The use of these activated imines was not tried in the reactions described in Scheme I; it was therefore surprising that using these special imines a β-lactam was formed indeed, being not a trans-β-lactam but a cis-β-lactam.

These reactions (without suitable metal compound, but with activated imine) are important, because, although in most cases trans-β-lactams are active as antibiotics, for some set of substituents of $R_1$, $R_{21}$, $R_3$ and $R_4$ only the cis-configuration of the β-lactam is active or may be converted into an active cis-β-lactam. Besides that, a procedure has been provided now to produce cis-β-lactams, similar to the synthetic method to produce trans-β-lactams (see EP-A-281177) with only one difference: no use of metal compound VIII' (see Scheme I). Such a streamlined procedure in synthesizing both trans- and cis-β-lactams gives many advantages, for instance regarding the use of cheap and easily obtainable materials etc.

The reactions according to the invention (both the reaction starting from the alkali metal enolate (II) and the one-pot process starting from the $R_2'R_3'$N-glycine ester (V) and the alkali metal base with the imine (IV)) producing the cis-β-lactams in a high yield and stereoselectivity, have been depicted in Scheme II:

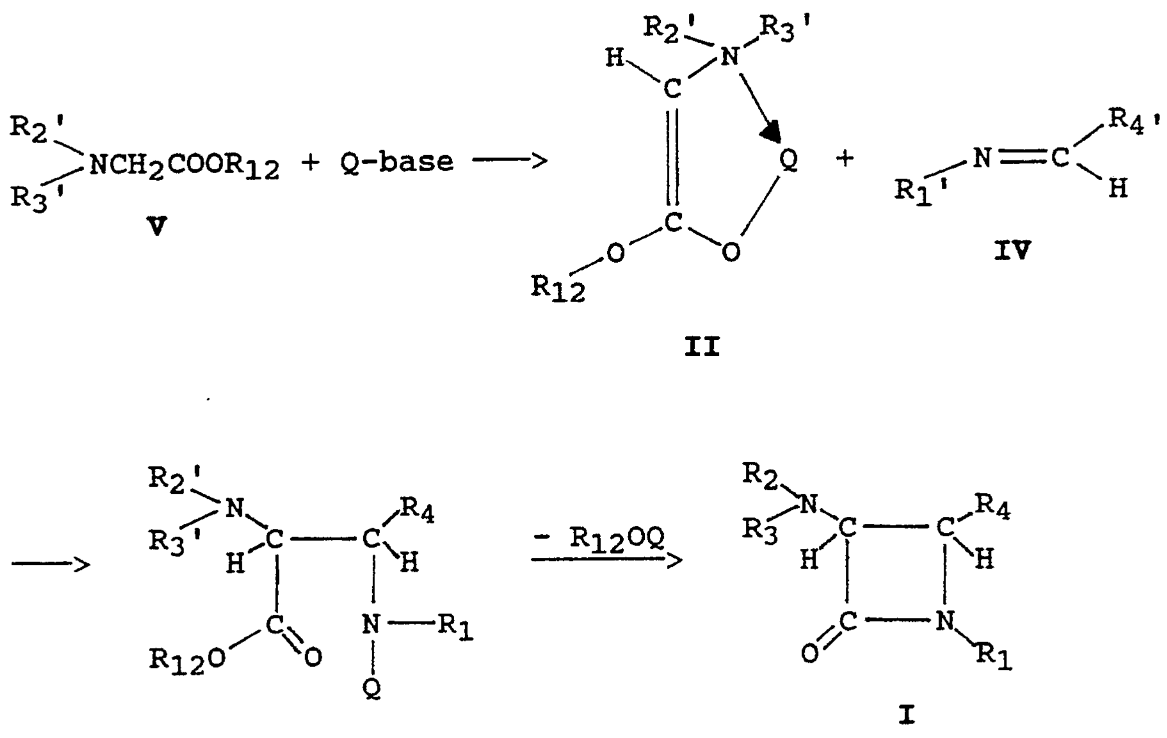


V

II

IV

I

**Scheme II**

The present invention accordingly provides a process for the preparation of a cis-β-lactam compound of formula I,

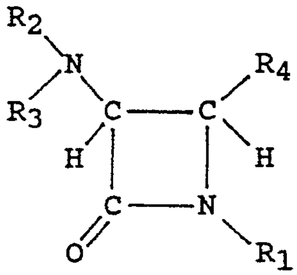

I

wherein
$R_2$ and $R_3$ are each, independently, hydrogen, or a (1-8C)alkyl, (6-18C)aryl or (1-8C)alkyl(6-18C)aryl group, each optionally substituted by a (1-8C)alkyl, (6-18C)aryl or (1-8C)alkyl(6-18C)aryl group, or the group

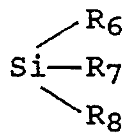

where $R_6$, $R_7$ and $R_8$ are each, independently, an optionally substituted (1-8C)alkyl, (6-18C)aryl or (1-8C)-alkyl(6-18C)aryl group,
or $R_2$ and $R_3$, together with the nitrogen atom to which they are attached, form a saturated ring with up to 6 ring atoms, each ring atom being optionally substituted by a (1-8C)alkyl, (6-18C)aryl or (1-8C)alkyl(6-18C)-arylgroup;
$R_1$ is an optionally substituted (6-18C)aryl, (1-8C)alkyl(6-18C)aryl or heterocyclic group, or is

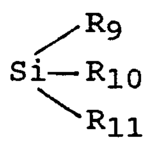

where $R_9$, $R_{10}$ and $R_{11}$ are each, independently, an optionally substituted (1-8C)alkyl, (6-18C)aryl or (1-8C)-alkyl(6-18C)aryl group;
$R_4$ is hydroxy, halogen, sulphonyl or an optionally substituted (1-8C)alkyl, (1-8C)alkoxy, (2-8C)alkenyl, (2-8C)alkynyl, (6-18C)aryl, (1-8C)alkyl(6-18C)aryl or heterocyclic group or is

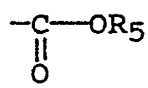

where $R_5$ is (1-8C)alkyl, or $R_4$ is

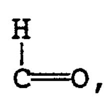

or

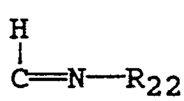

where $R_{22}$ is an optionally substituted (1-8C)alkyl, (6-18C)aryl, (1-8C)alkyl(6-18C)aryl or heterocyclic group, or $R_{22}$ is

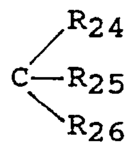

where $R_{24}$, $R_{25}$ and $R_{26}$ are each, independently, hydrogen or an optionally substituted (1-8C)alkyl, (1-8C)-alkoxy, (6-18C)aryl, (6-18C)aryloxy, (1-8C)alkyl(6-18C)aryl, (1-8C)alkoxy(6-18C)aryl or heterocyclic group, or $R_4$ is an optionally substituted heterocyclic group, which process comprises reacting a compound of formula II,

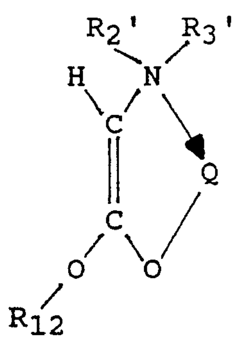

II

wherein
$R_{12}$ is a (1-8C)alkyl, (4-8C)cycloalkyl, (6-18C)aryl or (1-8C)alkyl(6-18C)aryl group, each optionally substituted by a (1-8C)alkyl, (4-8C)cycloalkyl, (6-18C)aryl or (1-8C)alkyl(6-18C)aryl group, Q is an alkali metal, $R_2$' and $R_3$' are as defined for $R_2$ and $R_3$ respectively, with the proviso that when $R_2$ and $R_3$ are hydrogen, $R_2$' and $R_3$' form, together with the nitrogen atom to which they are attached, a ring of formula III

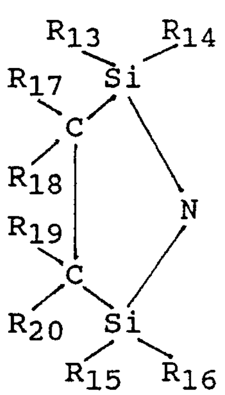

III

wherein
$R_{13}$, $R_{14}$, $R_{15}$, $R_{16}$, $R_{17}$, $R_{18}$, $R_{19}$ and $R_{20}$ are each, independently, a (1-8C)alkyl, (6-18C)aryl or (1-8C)alkyl-(6-18C)aryl group, or $R_{17}$, $R_{18}$, $R_{19}$ and $R_{20}$ are each, independently, hydrogen, or
$R_2$' and $R_3$' are

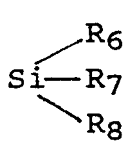

where $R_6$, $R_7$ and $R_8$ are as defined above, and $R_2$' and $R_3$' are the same, with an imine of formula IV,

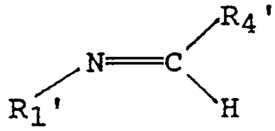

**IV**

where $R_1$' is as defined for $R_1$, with the proviso that when $R_1$ is hydrogen $R_1$' is

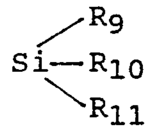

where $R_9$, $R_{10}$ and $R_{11}$ are as defined above,
which group is subjected, after the reaction of compounds II and IV, to acid or base hydrolysis,
and $R_4$' is as defined for $R_4$, with the proviso that when $R_4$ is

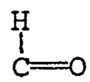 , $R_4$'

is

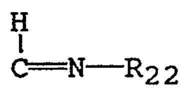 ,

which group is subjected, after the reaction of compounds II and IV, to acid hydrolysis, or $R_4$' is (1,2,O-isopropylidene)ethyl, which group is subjected, after the reaction of compounds II and IV, to oxidation, for instance with sodium periodate or lead tetraacetate,
and with the additional proviso that when $R_2$ and $R_3$ are both hydrogen, the process includes the additional step of converting the ring of formula III into $NH_2$ or the group

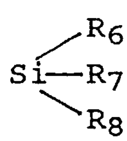

into hydrogen, by acid or base catalysed hydrolysis.

Particularly, Q in formula II is selected from lithium or sodium.

It has further been found that the preparation of cis-$\beta$-lactam compounds of formula I also can be carried out as a one-pot process which comprises reacting a glycine ester of formula V

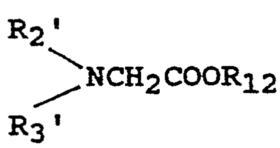

**V**

wherein

$R_2$', $R_3$' and $R_{12}$ are as defined above, with an imine of formula IV as defined above, in the presence of

an alkali metal base. This one-pot process forms a further aspect of the invention.

Particularly, the alkali metal base used is lithium diisopropylamide or sodium hexamethyldisilazane.

All the reactions are preferably carried out in inert, (weakly) polar solvents, for instance diethyl ether or tetrahydrofuran.

By a suitable, activated imine is meant an imine nitrogen substituted with (viz. $R_1'$ in formula IV) an electron-withdrawing group such as an aryl, an aralkyl, a heterocyclic group or a trisubstituted silyl group.

Optionally substituted as used herein means unsubstituted or substituted by any group generally present in organic compounds, such as halogen, hydroxy, alkyl, aryl, aralkyl, alkoxy, aralkoxy and heterocyclic groups.

As used herein, a heterocyclic group is a 5- or 6-membered heterocycle containing one or more heteroatoms, typically selected from O, N and S. The heterocycle can be fused to a second ring which may be heterocyclic or carbocyclic. Examples of heterocyclic groups within the present invention include thienyl, furyl, pyridyl, pyrryl, imidazolyl, pyridizinyl, pyrimidyl and pyrazinyl, all optionally substituted as defined above.

As used herein, the sulphonyl group is of the formula

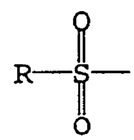

with R is (1-8C)alkyl, (6-18C)aryl or (1-8C)alkyl(1-8C)aryl, each optionally substituted.

The alkali metal enolate may be prepared by a method known *per se*, for instance from an alkali metal diisopropylamide and an N,N-dialkylglycine alkyl ester as described *in the* EP-A-281177.

The patent applications mentioned in this specification are incorporated herewith by reference.

The following Examples further illustrate the invention.

## Example I

### Synthesis of *cis*-3-(2,2,5,5-tetramethyl-1-aza-2,5-disilacyclopentyl)-4-substituted azetidinones

10 mmoles of n-butyllithium (6.67 ml of a 1.5 molar solution in hexane) was added to a stirred solution containing diisopropylamine (1.40 ml; 10 mmoles) and 25 ml of tetrahydrofuran at -70 °C. This reaction mixture was stirred for 10 minutes and then 2,2,5,5-tetramethyl-1-aza-2,5-disilacyclopentane-1-acetic acid ethyl ester (2.45 g; 10 mmoles) was added. The solution was stirred for 15 minutes at -70 °C and then N-(2-furyl-methylidene)trimethylsilylamine (1.67 g; 10 mmoles) was added. After 1 hour at -70 °C the reaction mixture was warmed up to room temperature and stirred for another hour, whereafter it was quenched with 30 ml of a saturated aqueous ammonium chloride solution. The water layer was extracted with diethyl ether. The diethyl ether extract was dried with sodium sulphate and concentrated *in vacuo* to give 3.48 g (95%) of the pure 2-azetidinone product as an off-white solid.

The $^1H$ NMR spectrum revealed that the product was a mixture of *cis*- and *trans*-isomers (*cis*:*trans* ratio 97:3).

The pure *cis*-product was obtained after recrystallization from diethyl ether/tetrahydrofuran as colourless crystals with the properties:

m.p. 126.5 °C.

$^1H$ NMR ($CDCl_3$): $\delta$ 7.38-7.37 and 6.35-6.28 (mp, 1H, mp, 2H, protons of the furyl group), 6.58 (br. s, 1H, NH), 4.76 (ddb, 1H, J = 4.6 and J = 1.3 Hz, H-C-CH-furyl), 4.69 ( db, 1H, J = 4.6 Hz, H-C-C-furyl), 0.69-0.50 (mp, 4H, $SiCH_2$), 0.06 and 0.00 (2 x s, 12H, $CH_3Si$).

$^{13}C$ NMR ($CDCl_3$): $\delta$ 170.88 (C=O), 151.14, 142.36, 110.52 and 109.34 (carbon atoms of the furyl group), 67.64 (C-CH-furyl), 53.68 (HC-C-furyl), 7.97 ($CH_2Si$), 0.58 and -0.11 ($CH_3Si$).

| Analysis : | | | | |
|---|---|---|---|---|
| Found : | C 52.07 | H 7.31 | N 9.45 | Si 18.8 |
| Calculated: | C 53.02 | H 7.53 | N 9.51 | Si 19.08 |

Table 1 shows the synthetic scheme for this cis-4-substituted azetidinone and two other representative examples.

**Table 1**

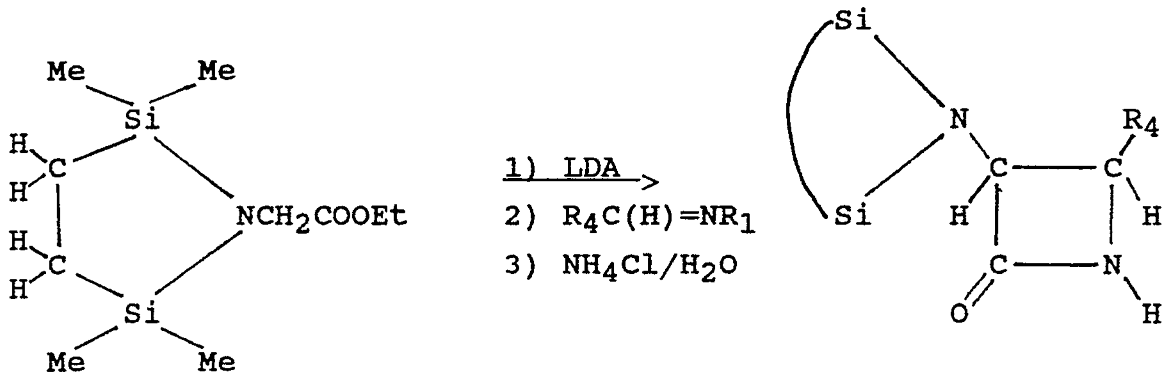


| | $R_1$ | $R_4$ | yield | cis/trans |
|---|---|---|---|---|
| 1. | $SiMe_3$ | furyl | 95% | 97/3 |
| 2. | $SiMe_3$ | phenyl | 99% | 96/4 |
| 3. | $SiMe_3$ | pyridyl | 92% | 91/9 |
| 4. | $SiMe_3$ | 2-thienyl | 99% | 94/6 |

**2. cis-3-(2,2,5,5-tetramethyl-1-aza-2,5-disilacyclopentyl)-4-phenyl-2-azetidinone:**
m.p. 136.5° C.
$^1$H NMR ($CDCl_3$): δ 7.41-7.22 (mp, 5H, protons of the phenyl group), 6.67 (br. s, 1H, NH), 4.81 (ddb, 1H, J = 5 and J = 1.6 Hz, C-CH-Ph) 4.77 (db, 1H, 3 = 5 Hz, HC-C-Ph), 0.62-0.50 (mp, 4H, $SiCH_2$), -0.02 and -0.15 (2 x s, 12H, $CH_3Si$).
$^{13}$C NMR ($CDCl_3$): δ 171.58 (C=O), 137.08, 128.19, 127.90 (carbon atoms of the phenyl group), 68.34 (C-CH-Ph), 59.62 (CH-C-Ph), 8.09 ($CH_2Si$), 0.50 and -0.18 ($CH_3Si$).

| Analysis | : | | | |
|---|---|---|---|---|
| Found | : | C 57.47 | H 7.99 | N 9.37 |
| Calculated: | | C 59.16 | H 7.94 | N 9.20 |

**3. cis-3-(2,2,5,5-tetramethyl-1-aza-2,5-disilacyclopentyl)-4-pyridyl-2-azetidinone:**
m.p. 98° C.
$^1$H NMR ($CDCl_3$): δ 8.58-8.55, 7.72-7.63, 7.38-7.34 and 7.26-7.16 (mp, 1H, mp, 1H, mp, 1H, mp, 1H, protons of the pyridyl group), 6.56 (br. s, 1H, NH), 4.90 (ddb, 1H, J = 5 Hz and J = 1.5 Hz, C-CH-pyridyl), 4.84 (db, 1H, J = 5 Nz, HC-C-pyridyl), 0.63-0.44 (mp, 4H, $CH_2Si$), -0.04 and -0.07 (2 x s, 12H, $CH_3Si$).
$^{13}$C NMR ($CDCl_3$): δ 170.88 (C=O), 157.04, 149.08, 155.75 and 122.84 (carbon atoms of the pyridyl group), 68.36 (C-CH-pyridyl), 60.62 (CH-C-pyridyl), 7.97 ($CH_2Si$), 0.59 and -0.06 ($CH_3Si$).

**4. cis-3-(2,2,5,5-tetramethyl-1-aza-2,5-disilacyclopentyl)-4-(2-thienyl)-2-azetidinone:**

Pale yellow solid, isolated yield 2.84 g (99%). The $^1$H NMR spectrum revealed that the product was a

mixture of cis- and trans-isomers (cis:trans ratio 94:6).

$^1H$ NMR ($CDCl_3$): cis, δ 7.30-7.24 (m, 1H, thienyl-H), 7.01-6.95 (m, 2H, thienyl-H), 6.74 (br.s, 1H, NH), 4.95 (d, 1H, J = 4.7, N-CH-CH-thienyl), 4.75 ( dd, 1H, J = 4.7 and 1.9 (N-CH-CH-thienyl), 0.66-0.64 (m, 4H, $SiCH_2CH_2Si$), -0.02, -0.03 (s, 6H, $Si(CH_3)_2$).

$^{13}C$ NMR ($CDCl_3$): cis, δ 170.71 (C=O), 140.93, 126.76, 126.54, 126.09 (thienyl-C), 68.18 (N-CH-CH-thienyl), 55.93 (N-CH-CH-thienyl), 8.02 ($SiCH_2CH_2Si$), 0.48, -0.23 ($Si(CH_3)_2$).

The pure cis-isomer was obtained as colourless crystals after one crystallization from benzene/diethyl ether (1:3 v/v). m.p. 117° C.

## Example II

### Synthesis of 3-dibenzylamino-4-phenyl-2-azetidinone

10 mmoles of n-butyllithium (6.67 ml of a 1.5 molar solution in hexane) was added to a stirred solution of diisopropylamine (1.40 ml; 10 mmoles) in 25 ml of tetrahydrofuran at 0° C. After stirring for 15 minutes N,N-dibenzyl glycine ethyl ester (2.83 g; 10 mmoles) was added at 0° C. The reaction mixture was stirred for another 15 minutes at room temperature and then N-(benzylidene)trimethylsilylamine was added. The mixture was stirred for 1 hour at room temperature and an additional 15 minutes at 50° C. After cooling to room temperature the reaction mixture was quenched with 30 ml of a saturated aqueous ammonium chloride solution. The water layer was extracted with diethyl ether. The diethyl ether extract was dried with sodium sulphate and concentrated in vacuo to afford 3.11 g (91%) of the pure 2-azetidinone product as an off-white solid.

The $^1H$ NMR spectrum revealed that the product was a mixture of cis- and trans-isomers (cis:trans ratio 84:16).

The pure cis product was obtained after recrystallization from diethyl ether as colourless crystals with the properties:

m.p. 80° C.

$^1H$ NMR ($CDCl_3$): δ 7.43-7.35, 7.28-7.18 and 7.05-7.00 (mp, 5H, mp, 6H, mp, 4H, protons of the phenyl group), 6.52 (br. s, 1H, NH), 4.78 (db, 1H, J = 5.3 Hz, CH-C-Ph), 4.54 (ddb, 1H, J = 5.3 Hz and J = 1.84 Hz, C-CH-Ph), 3.67 (db, 2H, J = 13.6 Hz, $CH_2Ph$), 3.51 (db, 2H, J = 13.6 Hz, $CH_2Ph$).

$^{13}C$ NMR ($CDCl_3$): δ 170.42 (C=O), 138.56, 137.40, 128.91, 128.52, 128.12, 127.79, 127.13 and 127.07 (carbon atoms of the phenyl groups), 73.69 (CH-C-Ph), 58.31 (C-CH-Ph), 55.53 ($CH_2Ph$).

## Claims

1. A process for the preparation of a cis-β-lactam compound of formula I

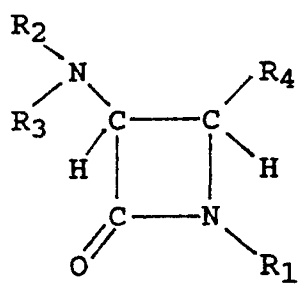

I

wherein

$R_2$ and $R_3$ are each, independently, hydrogen, or a (1-8C)alkyl, (6-18C)aryl or (1-8C)alkyl(6-18C)aryl group, each optionally substituted by a (1-8C)alkyl, (6-18C)aryl or (1-8C)alkyl(6-18C)aryl group, or the group

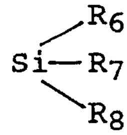

where $R_6$, $R_7$ and $R_8$ are each, independently, an optionally substituted (1-8C)alkyl, (6-18C)aryl or (1-8C)alkyl(6-18C)aryl group,
or $R_2$ and $R_3$, together with the nitrogen atom to which they are attached, form a saturated ring with up to 6 ring atoms, each ring atom being optionally substituted by a (1-8C) alkyl, (6-18C) aryl or (1-8C)-alkyl(6-18C)aryl group;
$R_1$ is an optionally substituted (6-18C) aryl, (1-8C)alkyl(6-18C)aryl or heterocyclic group, or is

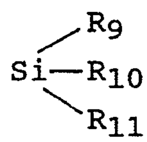

where $R_9$, $R_{10}$ and $R_{11}$ are each, independently, an optionally substituted (1-8C)alkyl, (6-18C)aryl or (1-8C)alkyl(6-18C)aryl group;
$R_4$ is hydroxy, halogen, sulphonyl or an optionally substituted (1-8C)alkyl, (1-8C)alkoxy, (2-8C)alkenyl, (2-8C)alkynyl, (6-18C)aryl, (1-8C)alkyl(6-18C)aryl or heterocyclic group or is

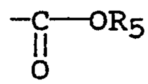

where $R_5$ is (1-8C)alkyl, or $R_4$ is

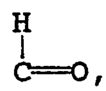

or

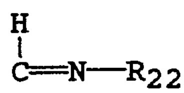

where $R_{22}$ is an optionally substituted (1-8C)alkyl, (6-18C)aryl, (1-8C)alkyl(6-18C)aryl or heterocyclic group, or
$R_{22}$ is

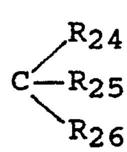

where $R_{24}$, $R_{25}$ and $R_{26}$ are each, independently, hydrogen or an optionally substituted (1-8C)alkyl, (1-8C)alkoxy, (6-18C)aryl, (6-18C)aryloxy, (1-8C)alkyl(6-18C)aryl, (1-8C)alkoxy(6-18C)aryl or heterocyclic group, or
$R_4$ is an optionally substituted heterocyclic group, which process comprises reacting a compound of formula II,

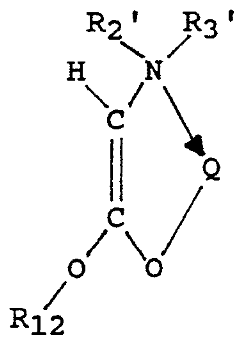

II

wherein

$R_{12}$ is a (1-8C)alkyl, (4-8C)cycloalkyl, (6-18C)aryl or (1-8C)alkyl(6-18C)aryl group, each optionally substituted by a (1-8C)alkyl, (4-8C)cycloalkyl, (6-18C)aryl or (1-8C)alkyl(6-18C)aryl group, Q is an alkali metal;

$R_2$' and $R_3$' are as defined for $R_2$ and $R_3$ respectively, with the proviso that when $R_2$ and $R_3$ are hydrogen, $R_2$' and $R_3$' form, together with the nitrogen atom to which they are attached, a ring of formula III

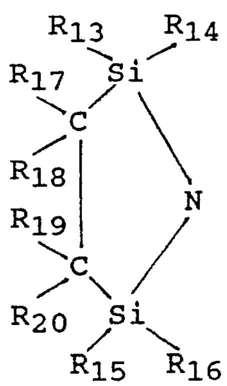

III

wherein

$R_{13}$, $R_{14}$, $R_{15}$, $R_{16}$, $R_{17}$, $R_{18}$, $R_{19}$ and $R_{20}$ are each, independently, a (1-8C)alkyl, (6-18C)aryl or (1-8C)-alkyl(6-18C)aryl group, or $R_{17}$, $R_{18}$, $R_{19}$ and $R_{20}$ are each, independently, hydrogen, or

$R_2$' and $R_3$' are

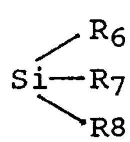

where $R_6$, $R_7$ and $R_8$ are as defined above, and $R_2$' and $R_3$' are the same, with an imine of formula IV,

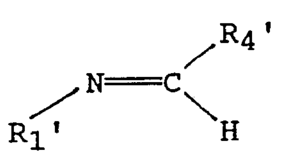

IV

where $R_1$' is as defined for $R_1$, with the proviso that when $R_1$ is hydrogen $R_1$' is

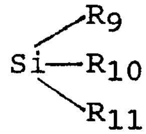

where $R_9$, $R_{10}$ and $R_{11}$ are as defined above, which group is subjected, after the reaction of compounds II and IV, to acid or base hydrolysis, and $R_4'$ is as defined for $R_4$, with the proviso that when $R_4$ is

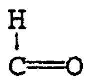 , $R_4'$ is 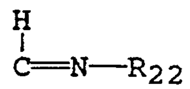

which group is subjected, after the reaction of compounds II and IV, to acid hydrolysis, or $R_4'$ is (1,2,O-isopropylidene)ethyl, which group is subjected, after the reaction of compounds II and IV, to oxidation, for instance with sodium periodate or lead tetraacetate, and with the additional proviso that when $R_2$ and $R_3$ are both hydrogen, the process includes the additional step of converting the ring of formula III into $NH_2$ or the group

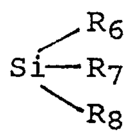

into hydrogen, by acid or base catalysed hydrolysis.

2. A one-pot process according to claim 1 for the preparation of a cis-$\beta$-lactam of formula I as defined in claim 1, which comprises reacting a glycine ester of formula V

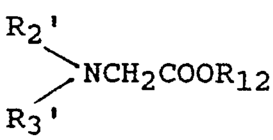

V

wherein $R_2'$, $R_3'$ and $R_{12}$ are as defined in claim 1, with an imine of formula IV as defined in claim 1, in the presence of an alkali metal base.

3. A process according to any one of the preceding claims, characterized in that the alkali metal is lithium or sodium.

4. A process according to claim 2, characterized in that the alkali metal base is lithium diisopropylamide or sodium hexamethyldisilazane.

5. A process according to any one of the preceding claims wherein, in formula I, $R_2$ and $R_3$ are benzyl or $R_2$ and $R_3$ together with the nitrogen atom to which they are attached form 2,2,5,5-tetramethyl-1-aza-2,5-disilacyclopentyl or $R_2$ and $R_3$ are hydrogen, $R_1$ is trimethylsilyl or hydrogen, and $R_4$ is phenyl, furyl, 2-pyridyl or 2-thienyl.

6. A process according to any one of the preceding claims, characterized in that the reaction is carried out in a (weakly) polar solvent.

7. A process according to claim 6, characterized in that the reaction is carried out in diethyl ether or tetrahydrofuran.

European Patent Office

# EUROPEAN SEARCH REPORT

Application Number

EP 91 20 0287

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 106, no. 19, 11th May 1987, page 655, abstract no. 156108j, Columbus, Ohio, US; N. OGUNI et al.: "Effect of addition of trialkylaluminums on the stereoselective synthesis of beta-lactum derivatives by the reaction of lithium enolate of esters with benzylideneaniline", & CHEM. EXPRESS 1986, 1(10), 579-82 * Abstract * | 1 | C 07 F 7/10<br>C 07 D 205/085 |
| A | CHEMICAL ABSTRACTS, vol. 105, no. 25, 22nd December 1986, page 746, abstract no. 226120n, Columbus, Ohio, US; C. WANG et al.: "A stereospecific synthesis of 1-benzyl-3-amido-4-substituted phenyl-2-azetidinones", & HUAXUE XUEBAO 1986, 44(3), 250-4 * Abstract * | 1 | |
| D,A | EP-A-0 281 177 (RIJKSUNIVERSITEIT UTRECHT) * Claims * | 1-7 | |
| D,A | EP-A-0 180 398 (THE REGENTS OF THE UNIVERSITY OF CALIFORNIA) * Claims; tables I-IV * | 1-7 | TECHNICAL FIELDS SEARCHED (Int. Cl.5) |
| P,A | EP-A-0 357 118 (RIJKSUNIVERSITEIT UTRECHT) * Claims * | 1-7 | C 07 F 7/00<br>C 07 D 205/00<br>C 07 D 401/00<br>C 07 D 405/00 |

The present search report has been drawn up for all claims

| Place of search | Date of completion of search | Examiner |
|---|---|---|
| The Hague | 16 April 91 | CHOULY J. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document